# EUROPÄISCHE PATENTSCHRIFT

(11) **EP 2 457 533 B1**
(45) Veröffentlichungstag und Bekanntmachung des Hinweises auf die Patenterteilung: **04.01.2017**
(21) Anmeldenummer: 11190283.9
(22) Anmeldetag: 23.11.2011
(51) Int. Cl.: A61B 34/30, A61B 1/06

(54) **Haltesystem für medizinische Instrumente**
Holder system for medical instruments
Système de retenue pour instruments médicaux

(30) Priorität: 24.11.2010 DE 102010052219
(43) Veröffentlichungstag der Anmeldung: 30.05.2012
(73) Patentinhaber: Karl Storz GmbH & Co. KG, 78532 Tuttlingen (DE)
(72) Erfinder: Abri, Omid, 14129 Berlin (DE); Schrader, Stephan, 14532 Kleinmachnow (DE); Irion, Klaus M., 78532 Tuttlingen (DE)

(56) Entgegenhaltungen:
- EP-A1- 1 728 482
- EP-A1- 2 283 790
- DE-A1-102010 027 248
- US-A1- 2005 060 974
- US-A1- 2006 171 693
- US-A1- 2009 103 174
- US-A1- 2010 274 078

## Beschreibung

Die Erfindung betrifft ein Haltesystem für medizinische Instrumente, mit mindestens einem Tragarm, an dem ein medizinisches Instrument festlegbar ist und mit mindestens einem Gelenk zur Positionierung des Tragarmes und/oder des medizinischen Instruments.

Derartige Haltesysteme sind bei der Durchführung chirurgischer Eingriffe häufig erforderlich, um medizinische Instrumente verschiedenster Art, wie beispielsweise Retraktoren, Endoskope oder Kameras über einen längeren Zeitraum in einer bestimmten Position zu halten. Durch die gelenkige Ausgestaltung des Haltesystems ist es für den Chirurgen möglich, das von dem Tragarm gehaltene Instrument exakt zu positionieren und die eingestellte Position des Haltesystems durch Arretieren beziehungsweise Fixieren des Gelenks bzw. der Gelenke des Tragarms zu fixieren.

Eine gattungsgemäßes Haltesystem ist beispielsweise aus der deutschen Patentanmeldung DE 195 21 060 A1 bekannt. Dieses bekannte Haltesystem besteht aus mehreren Tragarmen, welche gelenkig miteinander verbunden sind. Am distalen Ende des Haltesystems wir ein medizinisches Instrument befestigt bzw. arretiert. Die Zuführungen und Zuleitungen sind separat außerhalb des Haltesystems geführt und schränken die Bewegungsfreiheit des Operateurs stark ein.

Ein weiteres gattungsgemäßes Haltesystem wird in dem deutschen Gebrauchsmuster DE 92 18 373 U1 beschrieben. Auch hier handelt es sich um ein Haltesystem mit mehreren Tragarmen, welche gelenkig miteinander verbunden sind. Ein medizinisches Instrument ist am distalen Ende des Haltesystems festlegbar. Die Zuleitungen der einzelnen Geräte, z.B. Anschlussleitungen werden zu einem gemeinsamen Versorgungsschlauch zusammengefasst und über die Länge der Arme teilweise im Inneren eines Arms geführt. Diese Führung des Versorgungsschlauches im Inneren des Arms zeigt den Nachteil, dass die Sterilisationsanforderungen nur noch sehr schwierig realisierbar sind. Zudem ist durch Kabelführung ein beachtlicher Leistungsverlust gegeben, der gerade bei zusätzlichen Schnittstellen enorm anwächst, was z.B. beim Einsatz einer Lichtquelle zu signifikanten Lichtverlusten führen.

In US 2010/0274078 A1 ist ein Endoskopmanipulator mit einer kompakten und leichten Struktur offenbart, bei dem an der Spitze eines manuell verstellbaren Gelenkarms ein Endoskop montiert werden kann. Die Spitze des Gelenkarms ist mit einer solchen Halterung versehen, dass das Endoskop in drei Freiheitsgraden motorisch bewegt werden kann.

Gemäß US 2009/0103174 A1, die als nächstliegender Stand der Technik betrachtet wird, umfasst ein chirurgisches Beobachtungsystem mit einem in drei Dimensionen bewegbaren Tragarm eine Mikroskop- und eine Endoskop-Objektiveinheit, die über einen Befestigungsabschnitt am Tragarm befestigt werden können. Das von einer separaten Lichtquelle erzeugte Beleuchtungslicht wird über einen Lichtleiter zugeführt.

Aus EP 1728 482 A1 ist ein selbsteinstellendes Operationslampensystem mit einer Operationslampe bekannt, die an einer beweglichen Halterung angebracht ist, und mit einer Kamera, die in einem festen Positionsverhältnis zur Operationslampe angeordnet ist. An der Kamera sind Infrarot-LEDs angeordnet.

Gemäß US 2005/0060974 A1 ist bei einem Rauchabsaugungssystem für einen Operationssaal an einem Gelenkarm ein Service-Kopf angeordnet, der eine chirurgische Anordnung aufweist, die eine elektrische Energieversorgung benötigt. Die chirurgische Anordnung kann eine Lichtquelle umfassen.

In US 2006/0171693 A1 ist ein Endoskop mit einer Lichtquelleneinheit offenbart, die eine Anordnung von LEDs umfasst, die auf einem thermisch leitfähigen Substrat befestigt sind. Die Lichtquelleneinheit ist in das proximale Ende des Endoskops integriert und direkt an die optischen Fasern, die in die Endoskopspitze verlaufen, gekoppelt.

Davon ausgehend liegt der Erfindung die Aufgabe zugrunde, ein Haltesystem für medizinische Instrumente der eingangs genannten Art zu schaffen, das die oben genannten Nachteile möglichst reduziert. Die Kabelführungen und Zuleitungen sollen dabei ohne Funktionseinschränkungen ebenso möglichst verbessert werden.

Die Lösung dieser Aufgabe ist erfindungsgemäß durch die Merkmale des Patentanspruches 1 gelöst. Vorteilhafte Weiterbildungen der Erfindung sind Gegenstand der abhängigen Ansprüche.

Durch diese erfindungsgemäße Ausbildung des Haltesystems sind weniger oder kürzere Zuleitungen zu dem zu haltenden medizinischen Instrument erforderlich, was auch mit einem mehr an Bewegungsfreiheitsgraden für den Chirurgen verbunden ist. Durch die Reduktion der Zuleitungen existieren auch weniger Stolpermöglichkeiten im Operationssaal, was die Sicherheit im Operationssaal merklich erhöht. Entsprechendes gilt auch für eine Realisierung des erfindungsgemäßen Haltesystem in interventionellen Räumlichkeiten, beispielsweise in einem Hals-Nasen-Ohren- oder einem Gynäkologie-Behandlungszimmer. Nachfolgend wird die Erfindung am Beispiel einer Anordnung in einem Operationssaal erläutert, wobei dies entsprechend auf eine interventionelle Räumlichkeit übertragbar ist.

Auch ist eine Integration mehrerer funktioneller Baugruppen direkt im Haltesystem dahingehend vorteilhaft, dass auf zusätzliche Gerätewagen, die oft während der Operation im Weg stehen, vermieden werden können. Der Operateur kann somit alle oder viele für eine Operation wichtige Funktionen direkt bedienen und muss den Operationstisch während der Operation nicht verlassen, da das Haltesystem sich in seinem unmittelbaren Handhabungsbereich befindet. Das erfindungsgemäße Haltesystem ersetzt damit zumindest teilweise zusätzliche Gerätewagen im Operationssaal. Durch die vorteilhafte Integration mindestens einer Baugruppe direkt im Haltesystem ist eine einfachere Bedienung der Komponenten, welche während einer Operation notwendig sind, möglich. Ein störendes Verlassen des Operationsfeldes ist damit erfindungsgemäß regelmäßig nicht mehr nötig. Die Sicherheit im Operationssaal wird damit erfindungsgemäß erhöht.

Kabeldefekte, welche bisher durch Gerätewagen, die beim Verschieben oftmals über im Weg liegende Kabel gerollt wurden, zerstört wurden, werden durch die Erfindung vermieden. Durch das erfindungsgemäße Haltesystem kommen die Zuleitungen, Kabel und Versorgungsschläuche direkt aus einem Tragarm des Haltesystems, so dass Kabel, welche sich bisher quer durch den Raum erstreckten wegfallen und somit die Bewegungsfreiheit für den Operateur nicht mehr einschränken. Neben der Bewegungsfreiheit, die durch die erfindungsgemäße Integration und der damit verbundenen Kabelführung im Haltesystem geschaffen wurde, wird auch Bewegungsfreiheit dahingehend geschaffen, dass eventuelle Gerätewagen zumindest anteilig weg fallen können, da die entsprechenden Baugruppen direkt im Haltesystem integriert sind und damit immer in der Nähe des Operationsfeldes sind.

Aufgrund herum liegender Kabel gab es bisher sehr viele Störeffekte aufgrund elektrischer und elektromagnetischer Kopplungen. Diese werden durch den Erfindungsgegenstand maßgeblich verringert. Eine gebündelte und definierte Anordnung der Zuleitungen im Inneren des Haltearms vermindert die Störanfälligkeit nachweisbar. Zudem können die Zuleitungen im Inneren des Haltesystems zusätzlich isoliert bzw. abgeschirmt werden und direkt auf kurzem Weg zu der Baugruppe im Haltesystem geführt werden.

Verfügt das Haltesystem über mehr als einen Tragarm, sind diese mit einem oder mehreren Gelenken verbindbar, so dass das Haltesystem in mehrere Freiheitsgrade bewegbar ist. Vorzugsweise wird die mindestens eine Baugruppe im distalen Bereich des Haltesystems bzw. auch im distalen Bereich des Tragarms integriert, um zusätzliche Kabelführungen/Zuleitungen zu vermeiden. Der distale Bereich des Haltesystems ist der Bereich, an welchem auch das medizinische Instrument befestigbar ist. Der proximale Bereich des Haltesystems ist der von dem distalen Bereich entfernt gelegene Bereich d.h. z.B. nahe der Decken-, Boden- oder Tischhalterung. Prinzipiell kann das Haltesystem deckengestützt oder Operationstisch gestützt sein sowie auch autonom befestigt oder realisiert werden. Der Befestigungspunkt des Haltesystems ist dabei typisch im proximalen Bereich angeordnet.

Vorteilhafterweise wird vorgeschlagen im Tragarm ein modulares Baugruppensystem zu integrieren. Dieses kann aus einer oder mehreren Baugruppen bestehen, welche insbesondere im Tragarm integriert sind, wobei jede einzelne Baugruppe wiederum erfindungsgemäß eine oder mehrere Funktionseinheiten aufweist. In einem bevorzugten Ausführungsbeispiel werden beispielhaft zwei Baugruppen im Tragarm integriert, wobei eine der beiden Baugruppen eine Lichtquelle aufweist, während die andere Baugruppe die Steuereinheit und eine Datenkommunikationseinheit aufweist. Selbstverständlich sind auch andere Kombinationen der Baugruppen bzw. der integrierten Einheiten in den Baugruppen im Rahmen der Erfindung möglich. Durch die modulare Bauweise kann erfindungsgemäß ohne großen Aufwand das Haltesystem auf die jeweilige Operation schnell angepasst und die Tragarme mit den entsprechenden Baugruppen bzw. der integrierten Einheiten in den Baugruppen aus- oder umgerüstet werden, so dass die notwendigen funktionellen Einheiten immer direkt im bereich des Operationsfeld sind. Aufgrund der gebündelten Kabelführung im Haltearm werden zudem mögliche mechanische, elektrische und/oder elektromagnetische Störeffekte unterbunden, die durch frei liegende Kabel bisher immer wieder entstehen.

Gemäß einer bevorzugten Ausführungsform der Erfindung wird vorgeschlagen, eine Lichtquelle in die Baugruppe des Tragarms zu integrieren. Der Vorteil einer integrierten Lichtquelle besteht darin, dass es bei einer Lichtquelle außerhalb des Haltesystems mit einer Zuleitung, welche innerhalb des Haltesystems integriert ist, einer weiteren Schnittstelle bedarf, welche zu signifikanten Lichtverlusten führt. Diese zusätzliche Schnittstelle ist mit einer integrierten Lichtquelle nicht notwendig, somit werden auch die Lichtverluste verringert. Auch sind die Übertragungsstrecken für das Licht typisch geringen was wiederum zu einer besseren Lichtausbeute führt. Als Lichtquelle wird eine LED-Lichtquelle verwendet. Hierdurch ist eine kleinere Baugröße möglich als bei der Verwendung anderer Lichtquellen, wie z.B. eine Xenon-Lichtquelle. Zudem wird bei der Verwendung einer LED-Lichtquelle weniger Wärme erzeugt bei einer hohen Leistungsdichte. Der hohe Wirkungsgrad von LED's ist dabei besonders vorteilhaft bei der Umsetzung bzw. Integration in ein erfindungsgemäßes Haltesystem.

Auch wird ergänzend vorgeschlagen, dass die Bilderfassungseinheit, welche im Tragarm integriert ist, vorteilhafterweise eine endoskopische Kamera enthält. Die endoskopische Kamera verfügt über mindestens einen Bildaufnehmer und ist vorteilhafterweise auch im distalen Bereich des Haltearms insbesondere entnehmbar integriert. Es wird bevorzugt die mechanische Schnittstelle zwischen dem Haltesystem und dem medizinischen Instrument durch die Kupplung "Kamera/medizinisches Instrument" gebildet. Ein Vorteil dieser Ausführung besteht darin, dass die Videosignalversorgung nun auch aus dem Haltearm kommt und somit keine störenden zusätzlichen seitlich abgehenden Kabel mehr notwendig sind. Dabei hat es sich als besonders vorteilhaft erwiesen, wenn die Kamera so integriert wird, dass ihr Bedienfeld in integriertem Zustand weiterhin bedienbar ist. Auch ist an dieser Ausführungsform vorteilhaft, dass keine separate Bedienung von entfernt stehenden Komponenten zum Beispiel auf einem Gerätewagen notwendig ist. Das Operationsfeld muss somit nicht verlassen werden. Mit dieser Ausführungsform werden die Stolpergefahren im OP-Saal merklich reduziert. Zudem kam es in der Vergangenheit immer wieder vor, dass Zuleitungen zur Bilderfassungseinheit im Weg lagen und durch das Verschieben von Gerätewagen o.ä. überrollt wurden und somit zerstört wurden. In diesem Fall stand das Bildsignal für den Operateur nicht mehr zur Verfügung und die Operation musste unterbrochen werden. Durch die erfindungsgemäße Integration der Bilderfassungseinheit und der Zuleitungen im Haltesystem fallen diese Nachteile weg. Zudem wird die Störanfälligkeit des Systems durch Verminderung herumliegender bzw. herumhängender optischen oder elektrischen Kabel und Zuleitungen verringert.

In einer bevorzugten Ausführungsform der Erfindung befindet sich im distalen Bereich des Arms des Haltesystems eine elektromechanische Schnittstelle, über welche ein Videoendoskop mit integriertem Bildaufnehmer koppelbar ist. Vorzugsweise handelt es sich bei dem Endoskop um ein kostengünstiges disposables Endoskop. Sowohl die elektrische Versorgung bzw. das elektronische Bildsignal als auch die mechanische Kopplung des Videoendoskops erfolgen über die elektromechanische Schnittstelle. Auch ist es denkbar das Licht über die Schnittstelle zu führen. Als besonders vorteilhaft hat es sich erwiesen die elektromechanische Schnittestelle so auszubilden, dass die mechanische Aufnahe für ein Endoskop röhrenförmig entweder als beidseitig offene Röhre oder als unten geschlossene Röhre, in der Art eines Reagenzröhrchens, ausgebildet ist. Dabei können bevorzugt auch mehrere röhrenförmig Schnittstellen zu einer Reihe angeordnete sein, was die Handhabung und die elektronische Anbindung verbessert. Gerade bei der Verwendung von einseitig unten geschlossenen Röhren ist die Möglichkeit geschaffen eine Desinfektionslösung einzubringen, die das zwischengelagerte Endoskop von infektiösen Verunreinigungen befreit.

Wird vorteilhafterweise eine Bilddarstellungseinheit insbesondere eine Bildprojektionseinheit in dem Tragarm integriert, können u.a. patientenspezifische Daten bzw. ein endoskopisches Bild in das Gesichtsfeld des Operateurs, vorzugsweise in Richtung der Patientenoberfläche insbesondere im Operationsbereich projiziert werden. Vorzugsweise wird ein DLP-Projektor (Digital Light Processing) als Bildprojektor eingesetzt und somit das Bild im Gesichtsfeld des Chirurgen auf den Patienten erzeugt.

In einer weiteren erfindungsgemäßen Ausführungsform werden einem Tragarm eine Kamera sowie die entsprechende dazugehörige Signalverarbeitung, gerade in Verbindung mit einer Bilddarstellungseinheit, integriert. Vorteil dieser Ausführungsform ist neben der deutlichen Verminderung der Kabel auch die operationsnahe Positionierung der Kamera, die somit ohne Qualitätsverluste dem Operateur die notwendigen Bildinformationen z.B. in Form eines Bildes durch die in das Haltesystem integrierte Bilddarstellungseinheit, liefern kann. Die Störanfälligkeit des Systems gegen elektrische und elektromagnetische Effekte wird verringert, Stolperfallen im Operationssaal werden eliminiert und die Gefahr eines plötzlichen Bildverlustes, aufgrund herumliegender Kabel über welche man während der Operation bisher leicht stolperte und damit eventuell zerstörte, werden verringert. Alle diese positiven Effekte führen zu einer Erhöhung der Sicherheit im Operationssaal bzw. während der Operation.

In einer weiteren Ausführungsform werden in einem oder mehreren Tragarmen des Haltesystems Steuereinheiten integriert. Hierbei sind sowohl Steuereinheiten gemeint, die direkt das Haltesystem selbst steuern, als auch Steuereinheiten, die Funktionen bzw. Komponente außerhalb des Haltesystems steuern können. Insbesondere die Steuerung anderer Operationsgeräte direkt vom Haltesystem hat sich bewährt. Ein weiterer Vorteil dieser Ausführung ist, dass durch die erfindungsgemäße zusätzliche Steuerung dieser Komponenten auch beim Ausfall einer Steuerung die andere Steuerung als Rückfall-Position dienen kann. Zudem kann der Operateur so auch Komponenten bedienen, welche sich räumlich getrennt vom Operationsfeld befinden, ohne den Patienten aus den Augen zu lassen. Das Bedienfeld der Steuereinheit wird bevorzugt direkt im Bereich oder am distalen Ende des Haltesystems angebracht, so dass eine direkte Bedienung möglich ist ohne das Operationsfeld zu verlassen.

In einer weiteren Ausführungsform der Erfindung wird im Tragarm ein Sensor zur Abstands-, Oberflächenabtastung und/oder Umgebungszustandserfassung integriert. Beispiele für einen solchen Sensor sind unter Anderen eine time-of-flight Kamera (TOF-Kamera) oder ein Triangulationssensor. Auch werden Ultraschallsensoren, IR-Sensoren oder videobasierte Sensoren zur Erfassung der Umgebung erfindungsgemäß vorgeschlagen. Hierdurch kann der Operationstisch mit Patienten und Instrumenten kontinuierlich und punkt-, linien- oder flächenhaft erfasst werden. Dadurch gelingt es erfindungsgemäß, neben der Lage der Instrumente, die Patientenlage und Patientenposition zu erfassen und im Bedarfsfall das Haltesystem oder das Instrument bzw. den Patienten in der Lage nachzuführen. Auch können erfindungsgemäß die Operationstischbewegungen sowie die Lage, insbesondere der mit dem distalen Ende des Haltesystems verbundenen medizinischen Instrumente, für operative Navigationszwecke erfasst werden. Die Sensoren können zudem verwendet werden, um Gestiken des Operateurs zu erfassen und als Steuersignale insbesondere für das Haltesystem oder anderen angeschlossene Geräte zu interpretieren. Durch die Erfassung der Patientenlage können insbesondere die Daten und Bilder des Projektors der Patientenlage nachgeführt werden. Die räumliche Erfassung des Patienten sowie der medizinischen Instrumente und deren Lage zueinander dienen der operativen Navigationsunterstützung und/oder der Überlagerung bzw. dem Matching von prä- sowie intraoperativ generierten Patienten- oder Navigationsdaten. Durch die Erfassung von Tischbewegungen relativ zum Haltesystem werden Kollisionen von dem Patient bzw. dem Operationstisch mit dem Haltesystem vermieden. Durch die Erfassung der Gestik des Operateurs kann das Haltesystem kontaktfrei und somit steril angesteuert werden. Auch können damit andere Operationskomponenten genutzt werden, ohne den Operationstisch und somit den Patienten verlassen zu müssen und ohne aufwändige störende Verkabelungen.

Erfindungsgemäß werden ein oder mehrere dieser Lagesensoren im distalen Bereich des Haltesystems integriert. Somit kann auch bei einer räumlichen Verdrehung des distalen Tragarms mit einem daran befestigten Endoskop relativ zur Befestigung des Haltesystems oder des Patienten oder Operationstisches als Bezugspunkt mit Hilfe des Ausgangssignals der Lagesensoren das endoskopische Bild softwaretechnisch aufgerichtet und damit lagerichtig dargestellt werden.

Auch wird erfindungsgemäß vorgeschlagen, die integrierte Kamera bzw. den integrierten elektronischen Bildaufnehmer im distalen Bereich des Haltesystems rotierbar zu lagern. Sind nun Lagesensoren im Haltesystem integriert und das Haltesystem wird räumlich verdreht, wird dies von den Lagesensoren erfasst. Mittels einer Steuereinheit wird anschließend ein Signal an den Antrieb der rotierbaren Kamera bzw. den Antrieb des elektronischen Bildaufnehmers weitergegeben, und die Aufrichtung des Bildes motorisch insbesondere elektromotorisch und damit nicht softwaretechnisch gesteuert. Ein Horizontalausgleich kann vorteilhaft mit diesen Ausführungsformen erzeugt werden. Vorteil dieser Ausführung ist, dass das endoskopische Bild auch bei Bewegungen der Halterung stets aufrecht gehalten wird und dadurch eine sehr sichere Handhabung bei wenig störungsbelastetem Haltesystem gegeben ist.

Weiterhin wird bei einer weiteren bevorzugten Ausführungsform der Erfindung vorgeschlagen, dass Bowdenzüge in dem oder den Tragarmen integriert sind. Das eine Ende der Bowdenzüge ist mit der Kamera bzw. dem Bildsensor verbunden, während das andere Ende an einem proximal liegenden Tragarm befestigt ist, wobei dieser mit dem Bowdenzug befestigte Tragarm eine feste räumliche Lage zumindest in einem Freiheitsgrad besitzt. Der Bowdenzug wird derart befestigt, dass die Kamera bzw. der Bildsensor bei einer räumlichen Verdrehung des distalen Tragarms, in welchem die Kamera bzw. der elektronische Bildsensor rotierbar gelagert ist, stets aufgerichtet bleibt. Hierzu ist insbesondere eine Übersetzungsmechanik vorgesehen, die lagespezifisch die Bewegung des Bowdenzugs auf die Halterung der Kamera bzw. des Bildsensors übersetzungskorrekt überträgt.

Gemäß einer Ausführungsform der Erfindung wird vorgeschlagen, dass medizinische Geräte (z.B. Spül-/Saugpumpe, HF-Schneid- oder Koagulationsgeräte, Laserschneid- oder Koagulationsgeräte, Ultraschallschneid- oder Koagulationsgeräte, Ultraschallliptotripter) zur Verwendung in der minimal invasiven Chirurgie direkt im Tragarm integriert werden. Auch bei dieser Ausführungsform ist vorteilhaft, dass die Kabel im Haltesystem integriert sind und zentral aus dem Haltesystem kommen und keine seitlich abgehenden Kabel existieren, die die Bewegungsfreiheit des Operateurs unnötig einschränken. Unnötige Gerätewagen, welche bisher die Geräte bereitgestellt haben und oft im Weg standen, fallen somit weg. Zudem war ein Verschieben der Gerätewagen immer mit einem großen Kraftaufwand verbunden und nicht selten wurden die Kabel, welche zu den einzelnen Geräten am Gerätewagen führen überrollt bzw. lagen im Weg herum und verhinderten das Verschieben des Gerätewagens. Auch ist es nicht selten passiert, dass beim Verschieben des Gerätewagens die Länge der Zuleitungen und Kabel nicht beachtet wurde und bei zu kurzen Kabeln das entsprechende medizinische Gerät aus dem Gerätewagen heraus fiel und dabei zerstört wurde. Auch ist an der integrierten Anordnung vorteilhaft, dass zusätzliche Wege, zur Bedienung der außerhalb des Operationsfeldes befindlichen Komponenten, wegfallen. Die Integration zusätzlicher Funktionen im Haltesystem führt zu einer Erhöhung der Sicherheit im Operationssaal. Dabei ist die Integration in das Haltesystem so ausgebildet, dass die Geräte aus den Tragarmen entnommen bzw. eingesetzt und damit auf einfache Weise einer Wartung, Reparatur oder einer Anpassung an den jeweiligen Operationstyp und damit den Operationsbedürfnissen zugeführt werden können.

Des Weiteren wird erfindungsgemäß vorgeschlagen, mindestens eine Kühleinheit im Haltesystem zu integrieren. Aufgrund der mitunter vielen Baugruppen oder Funktionseinheiten, welche im Haltesystem integriert werden, entsteht eine beachtliche Abwärme am und im Haltesystem. Es hat sich bewährt zusätzlich eine Kühleinheit im Haltesystem zu integrieren, so dass die entstehende Wärme, der im Haltesystem integrierten Komponenten, effizient abgeleitet werden kann. Ohne Kühleinheit käme es schnell zu einer schädlichen Aufheizung und damit ggfs. zu einem Ausfall der integrierten Komponenten. Dies würde während der Operation zu einer erzwungenen Pause führen, die mitunter das Leben des Patienten gefährdet.

In einer Ausführung der Erfindung wird vorgeschlagen, im Haltesystem eine insbesondere drahtlose Datenkommunikationseinheit zu integrieren. Ein kabelloser Datenaustausch mit Geräten, welche sich innerhalb aber insbesondere auch außerhalb des Operationsfeldes befinden, wird damit ermöglicht. Hierdurch wird die Anzahl der Zuleitungen, welche die Bewegungsfreiheit des Operateurs einschränken können weiter reduziert.

Gemäß der Erfindung weist das Haltesystem einen Antrieb auf. Dabei kann es sich um elektromagnetische, piezoelektrische, pneumatische und/oder hydraulische Antriebe handeln. Vorteil dieser Ausführung ist, dass definierte Positionen automatisch angefahren werden können. Dies ist insbesondere wichtig, wenn Baugruppen in das Haltesystem integriert werden und dieses damit ein enormes Gewicht aufweist. Eine "Von Hand-Bedienung" ist bei einem solch schweren Haltesystem nur noch mit Mühe möglich. Durch einen geeigneten Antrieb kann somit auch ein Haltesystem mit vielen integrierten Baugruppen und Komponenten ohne Probleme positioniert werden. Auch ist es durch einen entsprechenden Antrieb möglich das Haltesystem gewichtsneutral auszubilden und dadurch einem einfachen Positionieren durch Ergreifen und Verschieben zugänglich zu machen. Neben den zuvor genannten Antrieben ist auch ein Gewichtsausgleich mittels mechanischer Komponenten z.B. durch Gegengewichte möglich.

Erfindungsgemäß wird weiterhin vorgeschlagen, dass das Haltesystem eine Hohlstruktur aufweist. Vorzugsweise wird vorgeschlagen, dass sowohl der mindestens eine Tragarm als auch die Gelenke eine durchgängige Hohlstruktur aufweisen. Durch die Hohlstruktur des Haltesystems können die Zuleitungen und die notwendigen Kabel direkt durch das Haltesystem geführt werden. Es liegen somit dem Operateur keine Kabel mehr im Weg. Auch wird die Störanfälligkeit stark minimiert. Durch die Bündelung der Zuleitungen im Haltesystem wird auch die elektromagnetische Verträglichkeit (EMV) im Operationssaal außerhalb des Haltesystems verbessert und die Störungen während der Operation verringert. Dies wird zum einen durch die Verkürzung der Kabel- und Leitungslängen, aber auch durch die Abschirmung der Hohlstruktur durch das sie umgebende Metall des Haltessystems in der art eines Faradayschen Käfigs wie auch durch zusätzliche EMV-Maßnahmen wir Verdrillung von Kabeln, zusätzliche Abschirmungen, Erdungen usw. erreicht.

Gemäß einer weiteren Ausführungsform der Erfindung wird vorgeschlagen, innere Zuleitungen und Steckerkontakte, insbesondere für HF-Chirurgie-Geräte, für motorbetriebene Systeme sowie für Videokameras, im Haltesystem bzw. im Tragarm zu integrieren. Damit können zusätzliche Kabel vermieden werden und es wird eine zentrale Kabelführung ohne Einschränkung des Operateurs gewährleistet. Auch dies dient der Verbesserung der Integration der verschieden Kabel oder Leitungen z.B. in Form eines zentralen Daten- oder Steuerbusses oder einer zentralen Energieversorgung oder zentralen Versorgung mit Medien, und auch der elektromagnetischen Verträglichkeit.

In einer weiteren Ausführungsform der Erfindung wird im proximalen Bereich des Haltesystems ein Lüfter integriert. In Kombination mit der Hohlstruktur über die ganze oder den wesentlichen Teil der Länge des Haltesystems kann mit Hilfe des Lüfters Luft von distal angesaugt und über die Hohlstruktur zum proximalen Ende abgeführt werden. Somit wird eine aktive Kühlung des Haltesystems ermöglicht. Je mehr Baugruppen bzw. Einheiten im Haltesystem integriert sind, desto erforderlicher wird die Integration einer Kühleinheit bzw. eines Lüfters im Haltesystem, da der Ausfall einer im Haltearm integrierten Komponente, wegen Überhitzung zu lebensgefährlichen Situationen führen kann. Neben dem Vorsehen eines Lüfters hat sich auch das Versehen einer Fluidförderpumpe bewährt, die ein fluidförmiges Kühlmitte insbesondere destilliertes Wasser als Kühlmittel in einem Fluidleitungssystem fördert und hierdurch eine ausreichende Kühlung gewährleistet. Dieses Fluidleitungssystem erstreckt sich dabei insbesondere im Wesentlichen über die gesamte Länge des Haltesystems. Die Leitungen haben bei vergleichbarer Kühlleistung einen relativ kleinen Durchmesser und füllen somit die Hohlstruktur nur zum geringen Anteil aus, wodurch ausrechend Raum für andere Zuleitungen oder Kabel gegeben ist. Dieses Fluidleitungssystem wird bevorzugt in der Art eines einfachen Fahrzeugkühlsystems ausgebildet.

In einer weiteren Ausführungsform der Erfindung werden insbesondere mehrere passive Wärmeleitelemente innerhalb der Hohlstruktur angeordnet. Vorteilhafterweise kann damit zum Beispiel die Wärme der Lichtquelle innerhalb der Hohlstruktur nach proximal abgeleitet werden. Ausführungsformen dieser Wärmeleitelemente sind z.B. metallische Stäbe oder auch heat pipes, die innerhalb der Hohlstruktur des Tragarms integriert sind. In den Gelenkabschnitten sind auch flexible Litzendrähte, z.B. aus Kupfer, als Wärmeleitelemente möglich. An den proximal liegenden Tragarmen wird bevorzugt die Wärme flächenhaft an die Hüllelemente des Tragarms übertragen und von dort an die Umgebung abgeführt. Alternative bilden Teile der Wärmeleitelemente selbst Teile der Hüllelemente. Diese Ausführung der Erfindung hat den Vorteil, dass eine Überhitzung des Systems in weitem Maß vermieden wird.

Weiterhin wird erfindungsgemäß vorgeschlagen in einzelnen und bevorzugt allen Gelenken optische, magnetische und/oder mechanische Encoderelemente zu integrieren, um die Position des Tragarms erfassen zu können. Durch die Messwertgeber kann die aktuelle Lage oder Position des jeweiligen Tragarms und damit des Haltesystems bzw. des medizinischen Instruments, welches am distalen Ende des Haltesystems angebracht ist, bestimmt werden. Dies erleichtert die Lagebestimmung einer möglichen Navigation erheblich. Vorzugsweise werden lineare Kodierer verwendet. Hierbei können Kodierer verwendet werden, die inkrementale, zu zählende oder auch absolute Maßverkörperungen als Strichmuster, Magnetisierung oder Kontakte aufweisen. Die Magnetfeldmodulation kann im Falle einer Dauermagnetisierung mittels AMR-, GMR, Hallsensoren oder induktiver Sensoren ausgewertet werden. Vorteil dieser Ausgestaltung ist, dass mit Hilfe des Ausgangssignals des Kodieres ein damit ausgerüstetes Haltesystem reproduzierbare Bewegungen ausführen kann. Wird erfindungsgemäß ein Absolutwertgeber verwendet, d.h. jeder Position wird ein eindeutiges Signalmuster zugeordnet, ist es möglich dass das Haltesystem auch nach dem Abschalten wieder in eine definierte Ausgangsposition bzw. Referenzposition fahren kann.

In einer Ausführungsform der Erfindung ist es somit möglich mit Hilfe der integrierten Sensorik und der entsprechenden Encoderelemente Preset-Positionen anzufahren. Durch die räumliche Erfassung des Operationsumfeldes und der Patientenlage ist es damit möglich Kollisionen zu vermeiden.

Erfindungsgemäß wird weiterhin vorgeschlagen, einen Kontrollmonitor im Haltesystem zu integrieren. Wird dieser vorteilhafterweise distal im Tragarm aufgenommen, kann dieser Monitor einfach beobachtet und die dargestellt Informationen einfach erfasst werden. Weiterhin kann bei Ausbildung des Monitors als Touchscreen zugleich eine Eingabe erfolgen und damit zielgerichtete Steuerung des Haltesystems mit seinen Baugruppen ermöglicht werden. Ein zusätzlicher Blick zu einem separaten Monitor zum Beispiel an der Wand des Operationssaales, welcher entfernt vom Operationsgebiet gelegen ist, ist somit nicht notwendig. Auch sind beispielhaft die Positionierung des Haltesystems und eine gleichzeitige Bildkontrolle erfindungsgemäß in der Blickrichtung möglich.

Bei dieser Erfindung handelt es sich um ein Haltesystem, welches neben der aus dem Stand der Technik bekannten Halte- und Positionierfunktion zusätzlich eine Funktionsplattform darstellt und mehrere Funktionseinheiten im Haltesystem integriert sind. Zudem werden die Zuleitungen direkt in dem Haltesystem geführt, um eine weitere Vereinfachung zu erhalten. Durch diese Ausgestaltung des erfindungsgemäßen Haltesystems ist ein sehr universelles Mittel zur Halterung von medizinischen Instrumenten gegeben, in welches über die Haltefunktion hinaus eine Vielzahl anderen Funktionen von medizinischen Geräten integriert ist, die gewöhnlich von auf Gerätewagen im Operationssaal angeordnet Geräten realisiert sind. Dies führt zu einem Haltesystem, das sehr funktionabel ist und sich im Hinblick auf die Betriebsicherheit im Operationssaal als sehr sicher erweist. Auch erweist es sich als sehr gut hanrlhahhar

Weitere Merkmale und Vorteile der Erfindung ergeben sich anhand der zugehörigen Zeichnungen, in denen Ausführungsbeispiele eines erfindungsgemäßen Haltesystems für medizinische Instrumente beispielhaft dargestellt sind. Die Erfindung ist nicht auf diese Ausführungsbeispiele beschränkt.

In den Zeichnungen zeigt:
- Fig. 1: eine Gesamtansicht eines erfindungsgemäßen Haltesystem
- Fig. 2: eine vereinfachte Ansicht des erfindungsgemäßen Haltesystem
- Fig. 3: ein Ausschnitt des distalen Endes des in Figur 2 dargestellten Haltesystem
- Fig. 4: ein Ausschnitt eines distalen Endes eines weiteren Ausführungsbeispieles des erfindungsgemäßen Haltesystem.

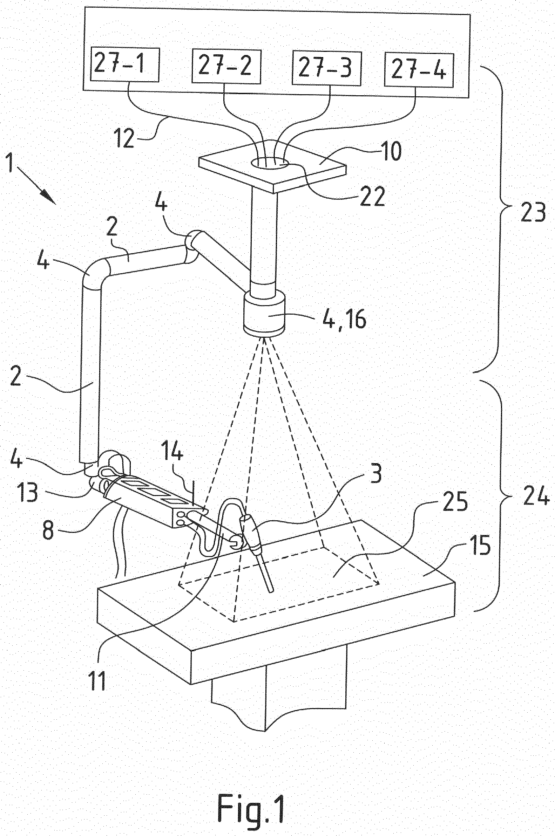

Die Fig. 1 zeigt ein erfindungsgemäßes Haltesystem für medizinische Instrumente.

Dieses Haltesystem 1 besteht im Wesentlichen aus mehreren Tragarmen 2, wobei die einzelnen Tragarme 2 mit Gelenken 4 relativ zueinander lösbar und arretierbar verschwenkbar miteinander verbunden sind.

Derartige Haltesysteme 1 sind bei der Durchführung chirurgischer Eingriffe häufig erforderlich, um medizinische Instrumente 3 verschiedenster Art, wie beispielsweise Retraktoren, Kameras, Mikroskope oder Endoskope, über einen längeren Zeitraum in einer bestimmten Position zu halten. Durch die gelenkige Ausgestaltung des Haltesystems 1 ist es für den Chirurgen möglich, das medizinische Instrument 3 in der gewünschten Lage zu positionieren und die eingestellte Position der Haltesystem 1 durch Arretieren des Gelenks 4 bzw. der Gelenke 4 zu fixieren. Neben der endoskopischen Chirurgie finden derartige Haltesysteme 1 auch in der offenen Chirurgie Anwendung.

Im Bereich des proximalen Endes 23 ist das Haltesystem 1 über eine als plane Platte ausgebildete Fixiereinheit 10, beispielsweise an der Decke, festlegbar. Das Haltesystem 1 kann über eine Fixiereinheit 10 auch am Operationstisch 15, an einer Wand des Operationssaales oder etwas Anderem festgelegt werden.

Am distalen Ende 24 weist der Tragarm 2 eine Instrumentenaufnahme 11 zur Aufnahme des über das Haltesystem 1 zu positionierenden medizinischen Instruments 3 auf. Wie aus Fig. 1 ersichtlich befindet sich die Instrumentenaufnahme 11 in unmittelbarer Nähe zum Operationstisch 15.

In dem Ausführungsbeispiel gemäß Figur 1 werden die Zuleitungen 12 der verschiedenen medizinischen Geräte 27-1, 27-2, 27-31, 27-4 welche in diesem Ausführungsbeispiel proximal im Deckenbereich insbesondere in einer Zwischendecke angeordnet sind, über eine sich durch das ganze Haltesystem 1 bzw. die Tragarme 2 erstreckende Hohlstruktur 22 geführt. Erfindungsgemäß sind Baugruppen mit verschiedenen Funktionseinheiten entsprechend einem oder mehreren abgesetzten medizinischer Geräte in einem der Tragarme 2 des Haltesystems 1 integriert angeordnet..

Erfindungsgemäß ist eine Bilddarstellungseinheit in Form einer Bildprojektionseinheit 16 in ein Gelenk 4 eines Tragarms 2 integriert. Die patientenspezifischen Daten oder Informationen auch in Form von Bildinformationen können so im Gesichtsfeld des Operateurs und hier direkt in den Operationsbereich 25 auf den Operationstisch 15 projiziert werden.

Am distalen Ende 24 des Haltesystems 1 ist eine Steckleiste 13 für Kabelverbindungen sowie eine Bedienkonsole mit Steuereinheit 8 zur Steuerung des Haltesystems 1 bzw. der Baugruppen oder auch externer Komponenten erkennbar. Die Bedienkonsole mit. Steuereinheit 8 verfügt erfindungsgemäß über Eingabe- und Anzeigeelemente sowie einer integrierten Lichtquelle 6. Die erfindungsgemäße Steckleiste 13 ermöglicht ein unkompliziertes und schnelles Verbinden des Haltesystems 1 mit weiteren medizinischen Geräten, welche zur Operation notwendig sind aber im Haltesystem 1 nicht integriert sind. Neben einer Steckleiste 13 können auch noch weitere bzw. andere Baugruppen im Tragarm 2 des Haltesystems 1 integriert sein.

Steuereinheiten 8 können so ausgebildet sein, dass eine drahtlose Datenkommunikation über die Sende- und Empfangsantenne 14 z.B. mit verschiedenen Operationsgeräten oder einem anderen Operationssaal möglich ist. Die Funkübertragung kann in Form einer, Bluetooth-Verbindung, einer Infrarotkommunikation oder Ähnliches erfolgen. Dabei soll das Bezugszeichen 14 eine Antenne darstellen, welche der Funkübertragung dient. Selbstverständlich kann die Antenne 14 auch in der Steuereinheit 8 integriert sein und ist somit von außen nicht sichtbar.

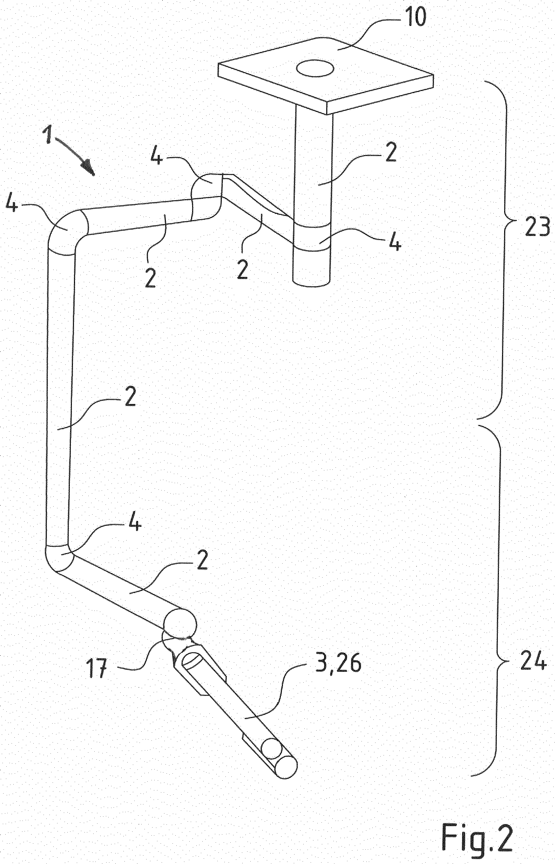

In der Figur 2 ist das Haltesystem aus Figur 1 vereinfacht dargestellt. Auch hier ist eine Fixiereinheit 10 erkennbar, mit welcher das proximale Ende 23 des Haltesystems 1 an der Decke befestigt werden kann.

Das Haltesystem 1 verfügt über mehrere Tragarme 2, die durch Gelenke 4 beweglich miteinander verbunden sind. Am distalen Ende 24 des Haltesystems 1 ist eine elektromechanische Schnittstelle 17 vorgesehen. Über diese Schnittstelle ist ein Videoendoskop 3; 26 mit integriertem Bildaufnehmer mit dem Haltesystem 1 mechanisch wie elektrisch und datentechnisch verbunden. Dieses Videoendoskop 26 ist erfindungsgemäß ein disposables und kostengünstiges Endoskop.

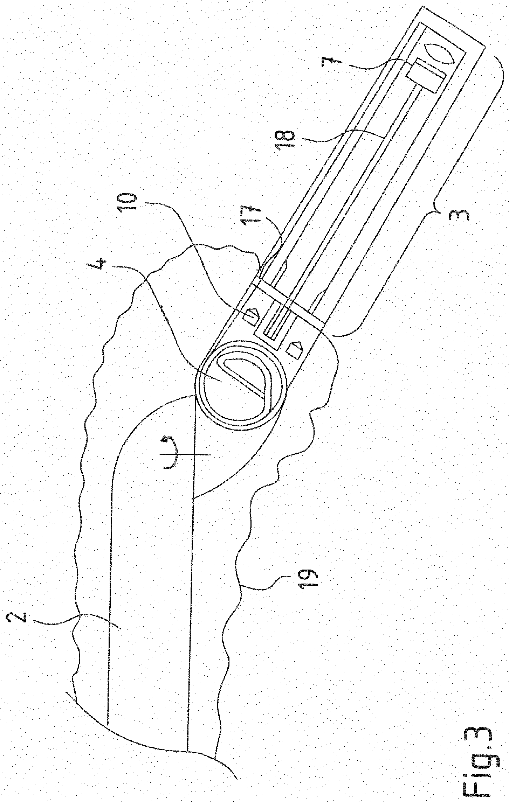

In Figur 3 ist das distale Ende 24 des Haltesystems 1 aus Figur 2 detaillierter dargestellt. Das Videoendoskop wird direkt über eine Schnittstelle 17 mit dem Tragarm 2 verbunden. Das Videoendoskop 3; 26 verfügt über eine als integrierten Bildaufnehmer ausgebildete Bilderfassungseinheit 7. Die elektrische Versorgung der Bilderfassungseinheit 7, welche sich im Videoendoskop 3,26 befindet, erfolgt durch eine Leiterplatte 18 mit proximalseitiger Steckerausführung. Die Bilderfassungseinheit 7 befindet direkt im medizinischen Videoendoskop 26 und wird über eine Leiterplatte 18 durch die Schnittstelle 17 mit dem Tragarm 2 verbunden. Im Tragarm 2 befindet sich die Lichtquelle, welche in diesem Ausführungsbeispiel als LED ausgebildet ist.

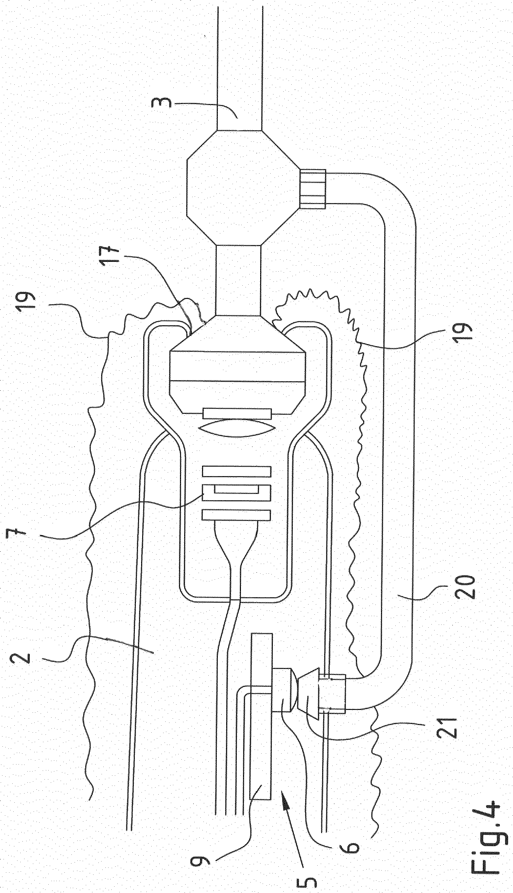

In den Ausführungsbeispielen in Figur 3 und Figur 4 verfügen die dargestellten Tragarme 2 jeweils über einen Sterilüberzug 19, welcher bis zum distalen Ende an die Schnittstelle 17 zwischen dem Tragarm 2 und dem medizinischen Instrument 3 reicht. Der Sterilüberzug 19 findet Anwendung, um die Sterilität insbesondere des Haltesystems 1 im Operationssaal zu gewährleisten.

In Figur 4 ist das distale Ende 24 eines weiteren Ausführungsbeispieles eines erfindungsgemäßen Haltesystems 1 dargestellt. Der Tragarm 2 ist an seinem distalen Ende 24 mit einer mechanischen Schnittstelle 17 ausgestattet. Diese Schnittstelle 17 verbindet den Tragarm 2 mit dem medizinischen Instrument 3, welches in diesem Ausführungsbeispiel ein Endoskop darstellt. Es handelt sich hierbei um eine Schnittstelle 17 zwischen dem Tragarm 2 und dem Endoskop 3. In der Schnittstelle 17 ist zudem eine Bilderfassungseinheit 7 mit verschiedenen Linsen, CCD und Auswerteschaltkreisen integriert.

Erfindungsgemäß ist wie in Figur 4 gezeigt im Tragarm 2 eine LED-Halbleiterlichtquelle 6 integriert. Diese Lichtquelle 6 mit zugeordneter Ansteuerung ist auf dem Kühlkörper 9 angeordnet und bildet zusammen eine Baugruppe 5. Der Kühlkörper 9 aus plattenförmigem Kupfer sorgt für einen ausreichenden Temperaturausgleich der Lichtquelle 6 und Verhinderung einer Überhitzung der Lichtquelle 6 und des Tragarms 2 bzw. der darin integrierten Funktionseinheiten bzw. Baugruppen.

Wie in der Figur 4 ersichtlich, verfügt erfindungsgemäß der Tragarm 2 über einen Lichtanschluss 21, welcher, wie in Figur 4 gezeigt, über einen Lichtleiter mit dem medizinischen Instrument 3, einem Endoskop, verbunden werden kann. Selbstverständlich sind auch andere bzw. mehrere Baugruppen 5 in einem Tragarm 2 integrierbar.

### Bezugszeichenliste

- 1: Haltesystem
- 2: Tragarm
- 3: medizinisches Instrument
- 4: Gelenk
- 5: Baugruppe
- 6: Lichtquelle
- 7: Bilderfassungseinheit
- 8: Steuereinheit
- 9: Kühleinheit
- 10: Fixiereinheit
- 11: Instrumentenaufnahme
- 12: Zuleitungen
- 13: Steckleiste
- 14: Antenne
- 15: Operationstisch
- 16: Projektor
- 17: Schnittstelle Tragarm/Videoendoskop
- 18: Leiterplatte
- 19: Sterilüberzug
- 20: Lichtleiter
- 21: Lichtanschluss
- 22: Hohl struktur
- 23: proximaler Bereich des Haltesystems
- 24: distaler Bereich des Haltesystems
- 25: Operationsbereich
- 26: Videoendoskop
- 27-1-27-4: Medizinisches Gerät

## Patentansprüche

1. Haltesystem (1) für medizinische Instrumente (3), nämlich für ein Endoskop, mit mindestens einem Tragarm (2), an dem das Endoskop festlegbar ist und mit mindestens einem Gelenk (4) zur Positionierung des mindestens einen Tragarmes (2) und / oder des Endoskops,
wobei
der Tragarm an seinem distalen Ende (24) mit einer mechanischen Schnittstelle (17) zum Verbinden des Tragarms (2) mit dem Endoskop ausgestattet ist, und im Tragarm (2) eine LED-Halbleiterlichtquelle (6) integriert ist, die mit zugeordneter Ansteuerung auf einem Kühlkörper (9) angeordnet ist, und zusammen mit der Ansteuerung eine Baugruppe (5) bildet, wobei der Tragarm über einen Lichtleiteranschluss (21) verfügt, welcher über einen Lichtleiter (20) mit dem Endoskop verbindbar ist, und im Tragarm (2) mindestens eine weitere Baugruppe integriert ist, die eine Bilderfassungseinheit (7) enthält, wobei die Bilderfassungseinheit mit verschiedenen Linsen, CCD und Auswerteschaltkreisen in die Schnittstelle (17) integriert ist, und der Tragarm (2) mittels elektromagnetischer, piezoelektrischer, pneumatischer und / oder hydraulischer Antriebe verstellbar ist.

2. Haltesystem (1) nach Anspruch 1, **dadurch gekennzeichnet, dass** die Bilderfassungseinheit (7) eine endoskopische Kamera enthält.

3. Haltesystem (1) nach einem der Ansprüche 1 oder 2, **dadurch gekennzeichnet, dass** das Haltesystem (1) eine Hohlstruktur (22) aufweist.

4. Haltesystem (1) nach Anspruch 3, **dadurch gekennzeichnet, dass** im proximalen Bereich des Haltesystems ein Lüfter oder eine Fluidpumpe angeordnet ist, der über die Hohlstruktur (22) des Haltesystems (1) Luft oder ein anderes Fluid von distal ansaugt und nach proximal fördert.

5. Haltesystem (1) nach Anspruch 3 oder 4, **dadurch gekennzeichnet, dass** wenigstens ein passives Wärmeleitelement innerhalb der Hohlstruktur (22) angeordnet sind, welche Wärme von distal nach proximal ableiten.

6. Haltesystem (1) nach Anspruch 5, **dadurch gekennzeichnet, dass** in dem Tragarm (2) wenigstens ein passives Wärmeleitelement als heat pipe ausgebildet ist.

7. Haltesystem (1) nach einem der vorstehenden Ansprüche 1 bis 6, **dadurch gekennzeichnet, dass** sich in oder am Gelenk (4) ein oder mehrere optische, magnetische und / oder mechanische Encoderelemente zur Erfassung der Position des oder der Tragarme (2) angeordnet sind.

8. Haltesystem (1) nach Anspruch 7, **dadurch gekennzeichnet, dass** sich in dem Gelenk (4) mit Encoderelement wenigstens ein Stellelement zur Positionierung des Tragarmes (2) angeordnet ist.

9. Haltesystem (1) nach einem der vorstehenden Ansprüche 1 bis 8, **dadurch gekennzeichnet, dass** im distalen Bereich des Arms des Haltesystems eine elektromechanische Schnittstelle für elektronische Anbindung und die mechanische Aufnahme eines Endoskops insbesondere eines Videoendoskops vorgesehen ist, wobei insbesondere die mechanische Aufnahme der elektromechanischen Schnittstelle röhrenförmig ausgebildet ist.

## Claims

1. Holding system (1) for medical instruments (3), to be precise for an endoscope, with at least one bracket (2) on which the endoscope can be secured, and with at least one joint (4) for positioning the at least one bracket (2) and/or the endoscope,
wherein the bracket is equipped, at its distal end (24), with a mechanical interface (17) for connecting the bracket (2) to the endoscope, and an LED semiconductor light source (6) is integrated in the bracket (2) and, with an associated control, is arranged on a heat sink (9) and, together with the control, forms an assembly (5), wherein the bracket has an optical waveguide attachment (21) which can be connected to the endoscope via an optical waveguide (20), and at least one further assembly is integrated in the bracket (2) and contains an image-recording unit (7), wherein the image-recording unit is integrated with various lenses, CCD and evaluation circuits in the interface (17), and the bracket (2) is adjustable by means of electromagnetic, piezoelectric, pneumatic and/or hydraulic drives.

2. Holding system (1) according Claim 1, **characterized in that** the image-recording unit (7) contains an endoscopic camera.

3. Holding system (1) according to either of Claims 1 and 2, **characterized in that** the holding system (1) has a hollow structure (22).

4. Holding system (1) according to Claim 3, **characterized in that**, in the proximal area of the holding system, a blower or a fluid pump is arranged that sucks in air or another fluid from the distal direction through the hollow structure (22) of the holding system (1) and conveys it in the proximal direction.

5. Holding system (1) according to Claim 3 or 4, **characterized in that** at least one passive heat conductor element is arranged inside the hollow structure (22) and diverts heat in the distal to proximal direction.

6. Holding system (1) according to Claim 5, **characterized in that** at least one passive heat conductor element in the bracket (2) is configured as a heat pipe.

7. Holding system (1) according to one of Claims 1 to 6, **characterized in that** one or more optical, magnetic and/or mechanical encoder elements are arranged in or on the joint (4) in order to detect the position of the bracket or brackets (2).

8. Holding system (1) according to Claim 7, **characterized in that** at least one actuator for positioning the bracket (2) is arranged in the joint (4) with encoder element.

9. Holding system (1) according to one of Claims 1 to 8, **characterized in that** an electromechanical interface for electronic linkage and mechanical reception of an endoscope, in particular a video endoscope, is provided in the distal area of the bracket of the holding system, wherein in particular the mechanical receiver of the electromechanical interface has a tubular shape.

## Revendications

1. Système de retenue (1) pour instruments médicaux (3), à savoir pour un endoscope, comprenant au moins un bras de support (2) sur lequel peut être fixé l'endoscope, et au moins une articulation (4) pour le positionnement de l'au moins un bras de support (2) et/ou de l'endoscope,
le bras de support étant muni au niveau de son extrémité distale (24) d'une interface mécanique (17) pour la connexion du bras de support (2) à l'endoscope, et une source de lumière à semi-conducteurs LED (6) étant intégrée dans le bras de support (2), laquelle est disposée avec une commande associée sur un corps de refroidissement (9) et forme, conjointement avec la commande, un module (5), le bras de support disposant d'un raccord à semi-conducteurs (21) qui peut être raccordé à l'endoscope par le biais d'un guide de lumière (20), et au moins un module supplémentaire étant intégré dans le bras de support (2), lequel comprend une unité de saisie d'image (7), l'unité de saisie d'image étant intégrée avec différentes lentilles, caméras CCD et différents circuits de d'analyse dans l'interface (7), et le bras de support (2) pouvant être réglé au moyen d'entraînements électromagnétiques, piézo-électriques, pneumatiques et/ou hydrauliques.

2. Système de retenue (1) selon revendication 1, **caractérisé en ce que** l'unité de saisie d'image (7) contient une caméra endoscopique.

3. Système de retenue (1) selon l'une quelconque des revendications 1 ou 2, **caractérisé en ce que** le système de retenue (1) présente une structure creuse (22).

4. Système de retenue (1) selon la revendication 3, **caractérisé en ce qu'**un ventilateur ou une pompe à fluide est disposé(e) dans la région proximale du système de retenue, lequel ou laquelle aspire de l'air ou un autre fluide distalement et le refoule proximalement par le biais de la structure creuse (22) du système de retenue (1).

5. Système de retenue (1) selon la revendication 3 ou 4, **caractérisé en ce qu'**au moins un élément thermoconducteur passif est disposé à l'intérieur de la structure creuse (22), lequel dévie la chaleur depuis une provenance distale dans la direction proximale.

6. Système de retenue (1) selon la revendication 5, **caractérisé en ce qu'**au moins un élément thermoconducteur passif est réalisé sous forme de tube échangeur de chaleur (Heat Pipe) dans le bras de support (2).

7. Système de retenue (1) selon l'une quelconque des revendications précédentes 1 à 6, **caractérisé en ce qu'**un ou plusieurs éléments de codage optiques, magnétiques et/ou mécaniques sont disposés dans ou sur l'articulation (4) pour détecter la position du ou des bras de support (2).

8. Système de retenue (1) selon la revendication 7, **caractérisé en ce qu'**au moins un élément de commande pour le positionnement du bras de support (2) est disposé dans l'articulation (4) avec l'élément de codage.

9. Système de retenue (1) selon l'une quelconque des revendications précédentes 1 à 8, **caractérisé en ce que** dans la région distale du bras du système de retenue est prévue une interface électromécanique pour la liaison électronique et la réception mécanique d'un endoscope, en particulier d'un endoscope vidéo, la réception mécanique par l'interface électromécanique étant notamment réalisée sous forme tubulaire.
